# EUROPÄISCHE PATENTSCHRIFT

(11) **EP 0 975 405 B1**
(45) Veröffentlichungstag und Bekanntmachung des Hinweises auf die Patenterteilung: **29.03.2006**
(21) Anmeldenummer: 98940072.6
(22) Anmeldetag: 17.04.1998
(51) Int. Cl.: B01D 15/00

(54) **VERWENDUNG VON AEROGELEN ALS ADSORPTIONSMITTEL**
USE OF AEROGELS AS ADSORPTION AGENTS
UTILISATION D'AEROGELS COMME AGENT D'ADSORPTION

(30) Priorität: 18.04.1997 DE 19716372; 07.05.1997 DE 19719395
(43) Veröffentlichungstag der Anmeldung: 02.02.2000
(60) Teilanmeldung: 06001672.2
(73) Patentinhaber: CABOT CORPORATION, Boston, Massachusetts 02210-2019 (US)
(72) Erfinder: SIEVERS, Werner, D-65929 Frankfurt am Main (DE); ZIMMERMANN, Andreas, D-64347 Driesheim (DE)
(74) Vertreter: Luderschmidt, Schüler & Partner GbR
(86) Internationale Anmeldenummer: PCT/EP1998/002283
(87) Internationale Veröffentlichungsnummer: WO 1998/047594

(56) Entgegenhaltungen:
- EP-A- 0 171 722
- WO-A-95/28220
- WO-A-96/19607
- WO-A-96/25850
- WO-A-96/25950

## Beschreibung

Die Erfindung betrifft Verfahren zur Isolierung von organischen Verbindungen aus wassingen Hussigkeiten.

Zur Reinigung von Gasen und Flüssigkeiten wie auch zur Stofftrennung wird eine Vielzahl von Adsorptionsmitteln eingesetzt, die aus einem Flüssigkeits-oder Gasgemisch einzelne Verbindungen oder Gruppen von Verbindungen adsorbieren und so eine Abtrennung ermöglichen. In der Regel werden heterogene Adsorptionsrrittet, insbesondere feste Adsorptionsmittel zur Reinigung von Flüssigkeiten und Gasen eingesetzt. Beispiele für bekannte Adsorptionsmittel sind Aktivkohle und polymere Adsorptionsmittel.

Mit Aktivkohle können eine Vielzahl von organischen und anorganischen Verbindungen adsorbiert werden. Dabei kann die Hydrophobizitöt der Aktivkohle kaum variiert werden, um beispielsweise eine selektive Adsorption aus einem Gemisch von Verbindungen mit unterschiedlicher Hydrophobizität zu ermöglchen. So haben Aktivkohlen, die in der Ablutttechnik eingesetzt werden, eine gewisse Wasseraufnahmekapazität, welche die für andere zu adsorbierende Verbindungen zur Verfügung stehende Adsorptionskapazität vermindert. Diese Wasseraufnahmekapazität kann nicht nur bei wasserdampfhaltigen oder wasserdampfgesättigten Abgasen nachteilig sein, sondern auch bei wasserdampfregenerierten Aktivkohleanlagen eine wichtige Rolle spielen. Bei der Regenerierung der Aktivkohleanlagen mit Wasserdampf muß nach dem Dämpfen ein zeitintensiver Trocknungsschritt eingefügt werden, in dem die feuchte Aktivkohle, meist mit Umgebungsluff. getrocknet wird. Somit ist die Regenerierung der Aktivkohle zeit- und arbeitsaufwendig. Bei der Verwendung von Aktivkohle oder Aktivkoks als Adsorptionsmittel bei der Lösemitteladsorption aus Abgasen, insbesondere in Anlagen zur Abluftreinigung, müssen die Adsorber in inerter Atmosphäre regeneriert werden. Bei der Adsorption und Desorption brennbarer Lösemittel kann es sonst zur Bildung von "Hot Spots", d.h. Glimmnestern, kommen, die den Abbrand der gesamten Adsorptionsanlage verursachen können. Deshalb wird als Desorptionsmedium neben Wasserdampf meist Stickstoff eingesetzt. Zudem sind oft zusätzliche Sicherheitseinrichtungen erforderlich, wie Vorrichtungen zum gezielten Fluten der Anlage mit Löschwasser im Brandfall. Derartige Sicherheitsvorkehrungen sind sehr kostspielig. Für bestimmte Adsorptionsaufgaben kann Aktivkohle nicht eingesetzt werden. So können Aktivkohlen in der Feinreinigung von pharmazeutischen Wirkstoffen und Produkten nicht verwendet werden, da Aktivkohlen nicht unerhebliche Aschebestandteite enthaHen, die nur teilweise durch kostenintensive Verfahren, wie einen Säureaufschluß, reduziert werden können.

Bei der Aufarbeitung von pharmakologischen Wirkstoffen wird daher bei Anwendung von Adsorptionsverfahren als produktschonendem Verfahren mit polymeren Adsorptionsmitteln gearbeitet. Polymere Adsorptionsmittel sind in der Regel hochvemetzte Copotymerisate, beispielsweise auf Basis von Styrol/Divinylbenzol. Diese polymeren Adsorptionsmittel werden in weitem Umfang bei der Reinigung von biologisch und chemisch hergestellten Arzneiwirkstoffen eingesetzt. Polymere Adsorptionsmittel quellen in Abhängigkeit vom verwendeten Lösemiffel unterschiedlich stark, so daß dieser gegebenenfalls erhöhten mechanischen Belastung bei der Dimensionierung der entsprechenden Apparate Rechnung getragen werden muß. Zudem sind polymere Adsorptionsmittel teuer. Die Hydrophobizität polymerer Adsorptionsmittel ist nur in relativ engen Grenzen einstellbar.

EP-A-0 171 722, WO-A-96/19607 und DE-A-3 937 863 beschreiben die Verwendung von Aerogelen als Adsorptionsmittel. WO-A-96/25950 betrifft die Verwendung von anorganischen Aerogelen in der Pharmazie bei der Beladung Freisetzung mit bzw. von Wirkstoffen. Z. B. wird die Beladung hydrophiler oder hydrophober Aerogele mit Wirkstoffen aus organischen Lösungsmitteln gezeigt.

Aufgabe der vorliegenden Erfindung ist die Bereitstellung von Adsorptionsmitteln, die insbesondere zur Behandlung von Abgasen, wie auch zur Reinigung von pharmazeutischen Wirkstoffen eingesetzt werden können und die vorstehend aufgeführten Nachteile von Aktivkohle und polymeren Adsorptionsmitteln vermeiden.

Erfindungsgemäß wird die Aufgabe durch ein Verfahren gemäß Anspruch 1 gelost.

Aerogele, die in der Regel hochporöse Materialien aus Silicium- oder Metalloxiden mit einer Dichte im Bereich von 70 bis 400 kg/m³ und einer inneren Obentöche von bis zu 1000 m²/g sind, werden Oberwiegend als thermische Isoliermaterialien eingesetzt, da sie sehr geringe Wärmeverlustfaktoren aufweisen.

Als Adsorptionsmittel sind Aerogele beispielsweise in einem Gasadsorptionselement beschrieben, in dem sie mit Aktivkohle und/oder Aktivtonerde gemeinsam in eine Matrix aus Papieren mit geringer Dichte, die hauptsöchlich aus anorganischen Fasern bestehen und aufeinandergeschichtet bzw. laminiert sind, eingebunden sind. Es werden Metallsitikat-Aerogele verwendet, die nicht modifiziert sind, wie z.B. in der DE-A 39 37 863 offenbart. Das Gasadsorptionselement wird übervviegend zum Binden von Luftfeuchtigkeit eingesetzt.

Es wurde gefunden, daß sich Aerogele hervorragend als Adsorptionsmittel in Gas- und Flüssigphasen einsetzen lassen.

Geeignete Aerogele sind beispielsweise in DE-A-43 16 540, DE-A-43 42 548, DE-A-44 22 912, DE-A-44 39 217, WO 96/22942, DE-A-195 250 21 und WO 98/05591 sowie in der noch nicht offengelegten deutschen Patentanmeldung 196 48 798.6 beschrieben.

Die nach den zitierten Druckschriften hergestellten Aerogele sind hydrophob. Durch eine Pyrolyse vorzugsweise bei 300 bis 600°C unter O₂-Atmosphäre lassen sich daraus hydrophile Aerogele herstellen, wie z.B. in der WO 96/26890 offenbart.

Die erfindungsgemäß eingesetzten Aerogele weisen sehr hohe innere Oberflächen von 100 bis 1000, vorzugsweise von 300 bis 700 m²/g, bestimmt durch BET-Messungen mittels Stickstoffadsorption, auf. Das Porenvolumen beträgt in der Regel 1,5 bis 3,5, vorzugsweise 2 bis 3 cm³/g. Ein Großteil der Porenradien liegt-dabei im Bereich von 1 bis 150, vorzugsweise im Bereich von 1 bis 30 und besonders bevorzugt im Bereich von 5 bis 20 Nanometer. Die Aerogele weisen dabei im Regelfall durchgängige Poren auf, d.h. alle Poren sind vom Partikelrand her zugänglich. Diese Eigenschaft ist bei Aktivkohle oder Aktivkoks nur durch einen zusätzlichen Verfahrensschritt, die thermische Aktivierung, zu erreichen.

Die Aerogele können in jede für die Anwendung als Adsorptionsmittel geeignete Form gebracht werden. So kann je nach Herstellung das Aerogel in Pulverform, als Formteil in beliebiger Form, vorzugsweise in Form von Strängen oder Stäbchen mit Durchmessern von 1 bis 4 mm und Längen von 3 bis 10 mm, oder als Granulat vorliegen. Die Aerogele können auch in Form von Tabletten oder Pellets eingesetzt werden. Für die Adsorption in der Flüssigphase werden vorzugsweise pulverförmige Adsorbentien eingesetzt, da aufgrund in der Regel geringerer Stoffübergangs- und Diffusionskoeffizienten kurze Diffusionswege angestrebt werden. Beim Einsatz in der Gasphase, etwa zur adsorptiven Gastrennung oder Abluftreinigung werden vorzugsweise Granulate, Tabletten, Pellets oder Formkörper der Aerogele eingesetzt, da so aufgrund eines hohen Lückenanteils Druckverluste gering gehalten werden können und kaum Staubprobleme, beispielsweise durch Austrag aus einer Adsorptionskolonne, auftreten. Die gewünschte Form der Aerogele kann direkt im Herstellungsverfahren erreicht werden, so daß auf nachträgliche Formgebungsprozesse verzichtet werden kann. Somit ist die Herstellung weniger aufwendig als beispielsweise das Formen von Aktivkohle, die zuerst gemahlen und dann in Form gepreßt werden muß.

Die Adsorptionseigenschaften der Aerogele können durch entsprechende Oberflächenmodifikationen, die einen hydrophilen oder hydrophoben Charakter der Aerogele bewirken, gezielt eingestellt werden. Eine Hydrophobierung erfolgt vorzugsweise durch Silylierung der Aerogele beispielsweise mit Trimethylchlorsilan. Durch Hydrophobierung kann die Wasseraufnahmekapazität der Aerogele sehr stark vermindert werden, so daß das Wasser die Oberflächen kaum benetzen kann. Hierdurch wird beispielsweise bei der Adsorption von wasserdampfhaltigen Abgasen die Adsorptionskapazität nicht durch Aufnahme von Wasser reduziert. Zudem ist bei einer Wasserdampfregenerierung der Aerogele kein zeitintensiver Trocknungsschritt erforderlich.

Bei der Lösemitteladsorption, insbesondere in Anlagen zur Abluftreinigung kann auf zusätzliche Sicherheitsvorkehrungen, wie sie beispielsweise bei Verwendung von Aktivkohle-Adsorptionsmitteln benötigt werden, verzichtet werden, da die Aerogele eine sehr hohe Temperaturstabilität aufweisen und unbrennbar sind. Somit können die Aerogele sehr vorteilhaft in Anlagen zur Abluftreinigung verwendet werden, wobei die hohen Anforderungen der TA Luft (TA = Technische Anleitung zur Reinhaltung der Luft), insbesondere in bezug auf Betriebssicherheit und Zuverlässigkeit, eingehalten werden. Es treten quasi keine Ausfaltzeiten der Reinigungsanlagen aufgrund technischer Störungen auf.

Neben der Lösemitteladsorption in Anlagen zur Abluftreinigung können die erfindungsgemäßen Aerogele für alle bekannten Adsorptionsanwendungen eingesetzt werden, in denen eine Adsorption aus der Gasphase erfolgen muß. Beispiele sind die Trennung von Gasgemischen und die Adsorption von Verunreinigungen aus Gasen. Bei den Verunreinigungen kann es sich beispielsweise um gasförmige Verunreinigungen, wie Stickoxide, Schwefeloxide. Kohlenmonoxid, Ammoniak oder organische Gase handeln. Es kann sich auch um verdampfte Flüssigkeiten oder im Gasstrom mitgerissene Flüssigkeibtröpfchen handeln.

Ist die Adsorptionskapazität der erfindungsgemäß eingesetzten Aerogele erschöpft, so können die Aerogele nach bekannten Verfahren regeneriert werden. Beispielsweise können die adsorbierten Stoffe thermisch entfernt werden. Auch eine Entfernung mit Hilfe anderer Gase, wie Wasserdampf ist möglich. Entsprechende Verfahren sind dem Fachmann bekannt.

Als "Gasphase" wird allgemein ein Stoffgemisch bezeichnet, das auf einem Gas oder Gasgemisch basiert. Das Gas oder Gasgemisch kann somit auch feste und insbesondere flüssige Bestandteile enthalten.

Die erfindungsgemäßen Aerogele können zudem als Adsorptionsmittel zur Adsorption aus einer Vielzahl von Flüssigphasen verwendet werden. Der Ausdruck "Flüssigphase" kann dabei homogene Lösungen. Emulsionen. Dispersionen, Flüssigkeiten, die Gase gelöst enthalten, und ähnliche Gemische einschließen. Eine Flüssigkeit dient dabei als Trögerphose, aus der Stoffe von den Aerogelen adsorbiert werden.

Es können somit Gase. Flüssigkeiten oder gelöste Stoffe oder auch Feststoffe aus Flüssigkeiten entfernt werden. Es handelt sich dabei um organische Stoffe. Insbesondere werden organische Stoffe, wie Kohlenwasserstoffe, insbesondere aromatische oder chlorierte Kohlenwasserstoffe entfernt. Die Adsorption erfolg aus Wasser oder wäßrigen Lösemittein. Die adsorbierten Verbindungen können dabei nach Trennung des Adsorptionsmittels von der Flüssigkeit durch geeignete Verfahren, beispielsweise durch Erhitzen. Auswaschen oder Eluieren freigesetzt werden. Die erfindungsgemäßen Aerogele zeigen dabei bei der Adsorption von organischen Wasserinhaltsstoffen hochselektive Adsorptionseigenschaften. Somit können sie sehr gut zur Reinigung von Industrieabwässern oder Laborabwössem eingesetzt werden.

Die erfindungsgemäBen Aerogele können neben der Reinigung von Flüssigkeiten auch zur Isolierung von organischen Verbindungen aus wässrigen Flüssigkeiten oder Flüssigkeitsgemischen eingesetzt werden. Hierbei ist das Ziel die Isolierung und Gewinnung von organischen Verbindungen, die teilweise stark verdünnt und neben vielen anderen Bestandteilen in Flüssigkeiten oder Flüssigkeitsgemischen vorliegen. Hierbei kann die hochselektive Adsorption an Aerogele vorteilhaft eingesetzt werden, um spezielle organische Verbindungen aus einer Vielzahl von gegebenenfalls ähnlichen organischen Verbindungen in einer Flüssigkeit zu isolieren.

Es werden dabei die organischen Verbindungen aus wäßrigen Flüssigkeiten isoliert. Es handelt sich insbesondere um Agrochemikalien oder pharmazeutische Wirkstoffe, die aus den bei ihrer Herstellung anfallenden Mutterlaugen isoliert werden. Beispiele für derartige Agrochemikalien sind Wirkstoffe mit herbiziden, fungiziden oder insektiziden Eigenschaften.

Bei der Herstellung von pharmazeutischen Wirkstoffen bilden die Kosten der Aufarbeitung oft einen erheblichen Anteil an den Gesamtkosten. Ein mikrobiologisch hergestelltes Produkt muß beispielsweise meist in mehreren Schritten so produktschonend wie möglich aufgearbeitet wenden. Die Aufarbeitung beinhaltet dabei die Abtrennung und Reinigung des pharmazeutischen Wirkstoffs aus der Fermentationslösung. Bei der Herstellung der Mehrzahl aller pharmazeutischen Wirkstoffe sind eine oder mehrere adsorptive Reinigungsschritte notwendig.

Beispiele für pharmazeutische Wirkstoffe sind Antibiotika, die aus den bei ihrer Herstellung anfallenden Fermentationslösungen gewonnen werden. Insbesondere werden die Aerogele zur Reinigung und Abtrennung des Antibiotikums Cephalosporin C (CPC) eingesetzt.

Durch die hohe Selektivität der erfindungsgemäßen Aerogele für die pharmazeutischen Wirkstoffe, insbesondere CPC, kann das Aufarbeitungs-und Reinigungsverfahren deutlich vereinfacht und beschleunigt werden. Die Erfindung betrifft auch die für die vorstehenden Anwendungen in Frage kommenden Reinigungs- und Isolierungsverfahren.

In einem Verfahren zur Reinigung von Gasen, werden die zu reinigenden Gase mit Aerogelen die nicht als Gemisch mit Aktivkohle oder Aktivtonerde vorliegen, als Adsorptionsmittel für einen Zeitraum in Kontakt gebracht, der für eine Adsorption von in den Gasen enthaltenen Verunreinigungen ausreicht. Entsprechende Kontaktzeiten sind dem Fachmann bekannt, sie liegen vorzugsweise in einem Bereich von 0,001 bis 0,01 Sekunden.

Ein erfindungsgemäßes Verfahren zur Isolierung von organischen Verbindungen aus Flüssigkeiten ist dadurch gekennzeichnet, daß die die organischen Verbindungen enthaltenden Flüssigkeiten mit Aerogelen als Adsorptionsmittel für einen Zeitraum in Kontakt gebracht werden, der für eine Adsorption der organischen Verbindungen ausreicht, sodann die Aerogele von den Flüssigkeiten abgetrennt und anschließend die organischen Verbindungen von den Aerogelen getrennt werden. Die Trennung kann dabei nach den vorstehenden Verfahren erfolgen. Bei der Reinigung von Flüssigkeiten und der Isolierung von organischen Verbindungen aus Flüssigkeiten erfolgt die Behandlung mit den Aerogelen in Zeiträumen die dem Fachmann bekannt sind, vorzugsweise für einen Zeitraum von 1 bis 50 Sekunden.
Die Abtrennung der gewonnenen organischen Verbindungen von den Aerogelen kann dabei wie vorstehend beschrieben durch Elution mit einem Lösemittel, etwa einem organischem Lösemittel oder einer Salzlösung, erfolgen.

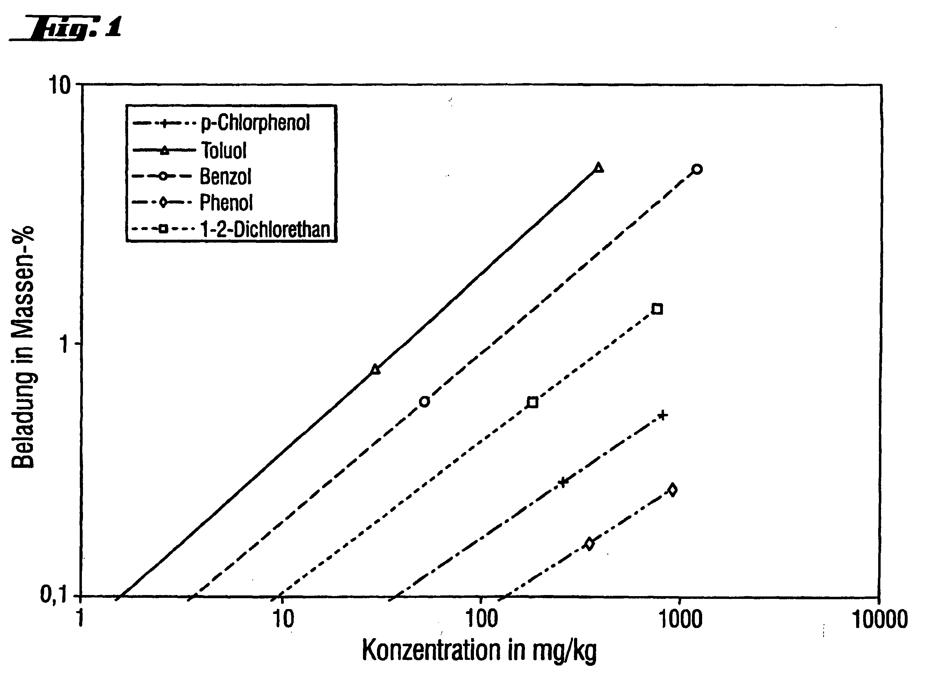

Die erfindungsgemäß verwendeten Aerogele werden vorzugsweise ohne weitere Trägerstoffe oder andere Adsorptionsmittel eingesetzt. Sie können jedoch auch in Kombination mit anderen Adsorptionsmitteln verwendet werden. Bei der Adsorption aus einer Gasphase werden sie auch nicht als Gemisch mit Aktivkohle oder Aktivtonerde eingesetzt, sofern es sich um nichtmodifizierte Aerogele handelt. Beispielsweise kann sich aber an eine Adsorptionsstufe mit Aerogelen eine Adsorptionsstufe mit Aktivkohle oder Aktivtonerde anschließen. Die weiteren Adsorptionsstufen können dabei zur weiteren Aufreinigung der Gase, Flüssigkeiten oder organischen Verbindungen eingesetzt werden. Adsorptionsmittel, die mit den erfindungsgemäßen Aerogelen kombiniert werden können, sind dem Fachmann bekannt. Die Erfindung wird nachstehend zusätzlich anhand von Beispielen in Verbindung mit der Zeichnung erläutert, die in
- Fig. 1: in einem Diagramm die Beladung von Aerogel mit unterschiedlichen organischen Verbindungen in Abhängigkeit von deren Konzentration in Wasser zeigt.

### Beispiel 1

### Adsorption von Wasserinhaltsstoffen

Um die selektive Adsorption von organischen Verbindungen aus Abwässern zu zeigen, wurden als Beispiele für die Gruppen der aromatischen und/oder chlorierten Kohlenwasserstoffe Benzol, Phenol, Toluol, 1,2-Dichlorethan und p-Chlorphenol ausgewählt. Diese Verbindungen wurden in unterschiedlichen Konzentrationen in Wasser bei 25°C mit Aerogel kontaktiert, wobei die erzielbare Beladung des Aerogels gemessen wurde. Dabei wurden die Versuche in einem Schüttelkolben ausgeführt. Hierzu wurden in dem Schüttelkolben 6 g hydrophobes Aerogel, das analog dem Beispiel der DE-A-43 42 548 hergestellt wurde (Teilchengröße 0,1 mm, spezifische innere Oberfläche ca. 500 m²/g), mit 0,1 Liter des zu reinigenden wäßrigen Gemisches in Kontakt gebracht. Über die Messung der Flüssigkeitskonzentration vor und nach der Adsorptionszeit, die maximal 2 Stunden bis zur Einstellung des Gleichgewichts betrug, konnte aus einer Massenbilanz die adsorbierte Stoffmenge berechnet werden. Die Ergebnisse sind in der Figur 1 dargestellt.

Die in der Figur 1 gezeigten Ergebnisse zeigen, daß Aerogele sehr gut zur Reinigung von Abwässern eingesetzt werden können, die organische Verunreinigungen enthalten.

### Beispiel 2 und Vergleichsbeispiele

### Adsorption eines Antibiotikums

Als Beispiel für die Aufarbeitung eines pharmazeutischen Wirkstoffs aus der bei seiner Herstellung anfallenden Fermentationslösung wurde die Adsorption des Antibiotikums Cephalosporin C (CPC) untersucht. CPC fällt bei der Herstellung in einer Konzentration von wenigen Gramm pro Liter Fermentationslösung an und muß aus dieser Lösung so schonend wie möglich abgetrennt werden. Dabei ist zu beachten, daß die wäßrige Fermentationslösung neben einer erheblichen Anzahl weiterer Aminosäuren, Salzen, Zuckern und anderen inhaltsstoffen als Nebenprodukte der Biosynthese Desacetyl-CPC (D-CPC) und Desacetoxy-CPC (DO-CPC) enthält. Für die Aufreinigung mittels Adsorption ist dabei von großer Bedeutung, daß nur das CPC, nicht aber D-CPC und DO-CPC adsorbiert werden. Die erfindungsgemäßen Aerogele können hierfür mit hoher Selektivität eingesetzt werden, d.h. es wird im wesentlichen nur CPC adsorbiert. Für die Untersuchung wurde das hydrophobe Aerogel aus Beispiel 1 eingesetzt.
Für die Untersuchungen wurde eine Fermentationslösung mit einem CPC-Gehalt von 10 g/l, D-CPC von 2 g/l und DO-CPC von 0,1 g/l eingesetzt. Es wurde insgesamt 1 Liter dieser Fermentationslösung über eine Festbettschüttung mit 100 g Aerogel gepumpt. Danach wurden die adsorbierten Stoffe mit einem Liter Isopropanol ausgewaschen. Die Analyse der Mengen an adsorbierten CPC, D-CPC und DO-CPC erfolgte durch eine HPLC-Messung. Zu Vergleichszwecken wurden als Adsorptionsmittel die Harze XAD 16 der Firma Rohm und Haas sowie SP 825 der firma Mitsubishi Chemicals eingesetzt.

Die Ergebnisse für die Gleichgewichtsbeladungen des Aerogels in g pro kg Aerogel und die Selektivitäten sind in der nachstehenden Tabelle 1 aufgeführt:

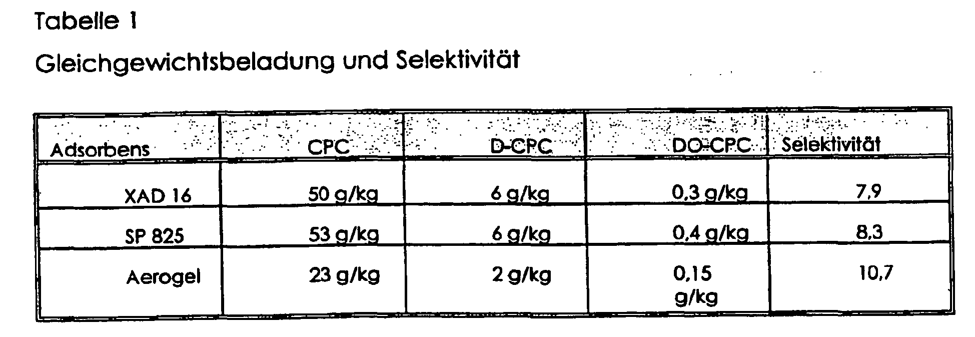

Die Selektivität des Aerogels ist um mehr als 20% besser als bei den Vergleichsadsorptionsmitteln, wodurch eine erheblich bessere Reinigung von CPC ermöglicht wird. Zudem wurde die Gleichgevvichtsbeladung des Aerogels bei unterschiedlich CPC-Konzentrationen gemessen. Bei einer CPC-Konzentration von 2,5 g/l betrug die Beladung 8 g/kg, bei 5 g/l 16,5 g/kg, bei 7,5 g/l 20 g/kg und bei 10 g/l 23 g/kg.

## Patentansprüche

1. Verfahren zur Isolierung, von organischen Verbindungen aus wässrigen Flüssigkeiten, **dadurch gekennzeichnet, dass** die organischen Verbindungen enthaltenden wässrigen Flüssigkeiten mit hydrophobierten Aerogelen als Adsorptionsmittel für einen Zeitraum in Kontakt gebracht werden, der für eine Adsorption der organischen Verbindungen ausreicht, sodann die Aerogele von den Flüssigkeiten abgetrennt und anschließend die organischen Verbindungen von den Aerogelen getrennt werden.

2. Verfahren nach Anspruch 1, **dadurch gekennzeichnet, dass** die organischen Verbindungen Agrochemikalien oder pharmazeutische Wirkstoffe sind, die aus den bei ihrer Herstellung anfallenden Mutterlaugen isoliert werden.

3. Verfahren nach Anspruch 2, **dadurch gekennzeichnet, dass** die pharmazeutischen Wirkstoffe Antibiotika sind, die aus Fermentationslösungen isoliert werden.

4. Verfahren nach einem der Ansprüche 1 - 3, **dadurch gekennzeichnet, dass** die Aerogele keine weiteren Trägerstoffe und andere Adsorptionsmittel enthalten.

## Revendications

1. Procédé pour l'isolement de composés organiques à partir de liquides aqueux, **caractérisé en ce que** les liquides aqueux contenant les composés organiques sont mis en contact avec des aérogels rendus hydrophobes en tant qu'agents d'adsorption pendant une période suffisante pour l'adsorption des composés organiques, les aérogels sont séparés des liquides et, consécutivement, les composés organiques sont séparés des aérogels.

2. Procédé selon la revendication 1, **caractérisé en ce que** les composés organiques sont des produits agrochimiques ou principes actifs pharmaceutiques qui sont isolés des eaux mères produites lors de leur fabrication.

3. Procédé selon la revendication 2, **caractérisé en ce que** les principes actifs pharmaceutiques sont des antibiotiques qui sont isolés à partir des solutions de fermentation.

4. Procédé selon l'une des revendications 1 à 3, **caractérisé en ce que** les aérogels ne contiennent pas de supports supplémentaires, ni d'autres agents d'adsorption.

## Claims

1. Process for the isolation of organic compounds from aqueous liquids, **characterised in that** the aqueous liquids containing the organic compounds are brought into contact with aerogels that have been rendered hydrophobic, as adsorption agents, for a period of time sufficient for adsorption of the organic compounds, after which the aerogels are separated from the liquids and then the organic compounds are separated from the aerogels.

2. Process according to claim 1, **characterised in that** the organic compounds are agricultural chemicals or pharmaceutical active ingredients which are isolated from mother liquors produced during their production.

3. Process according to claim 2, **characterised in that** the pharmaceutical active ingredients are antibiotics which are isolated from fermentation solutions.

4. Process according to one of claims 1-3, **characterised in that** the aerogels comprise no further carriers and other adsorption agents.
